# FASCICULE DE BREVET EUROPEEN

(11) **EP 1 201 228 B1**
(45) Date de publication et mention de la délivrance du brevet: **18.08.2004**
(21) Numéro de dépôt: 01402554.8
(22) Date de dépôt: 03.10.2001
(51) Int. Cl.: A61K 7/48, A61K 7/42

(54) **Composition cosmétique, comprenant un rétinoide et une silicone benzotriazole**
Kosmetische Zusammensetzung enthaltend ein Retinoid und ein Silicon Benzotriazol
Cosmetic composition containing a retinoid and a silicone benzotriazol

(30) Priorité: 30.10.2000 FR 0013938
(43) Date de publication de la demande: 02.05.2002
(73) Titulaire: L'OREAL, 75008 Paris (FR)
(72) Inventeur: Martin, Guénaelle, 75012 Paris (FR); Touzan, Philippe, 75012 Paris (FR)
(74) Mandataire: Renard, Emmanuelle

(56) Documents cités:
- EP-A- 0 609 963
- EP-A- 0 985 404
- US-A- 5 089 250

## Description

La présente invention a pour objet de nouvelles compositions cosmétiques et/ou dermatologiques, en particulier des compositions destinées au soin de la peau et comprenant, dans un milieu physiologiquement acceptable, un rétinoïde et une silicone benzotriazole.

Les compositions cosmétiques et/ou dermatologiques à base de rétinoïdes ont connu un important développement au cours de ces dernières années, en particulier pour le traitement de l'acné et des imperfections cutanées, en raison de la capacité des rétinoïdes à réguler la différenciation des kératinocytes, et pour la prévention et le traitement de certains signes du vieillissement cutané intrinsèque ou photo-induit, tels que la formation de rides et la perte de fermeté de la peau, en raison notamment de leur capacité à moduler la synthèse de collagène.

Parmi les dérivés de la famille des rétinoïdes, le rétinol, également connu sous le nom de vitamine A, présente un intérêt tout particulier. En effet, le rétinol est un constituant endogène naturel de l'organisme humain qui est bien toléré en application sur la peau jusqu'à des taux plus élevés que l'acide rétinoïque.

Toutefois, lorsqu'il est introduit dans une composition cosmétique ou dermatologique destinée à une application topique, le rétinol se dégrade rapidement, sous l'effet de la lumière, de l'oxygène, des ions métalliques, des agents oxydants, de l'eau ou en particulier sous l'effet d'une élévation de température. La dégradation thermique du rétinol a fait l'objet d'une étude publiée dans J. Soc. Cosm. Chem. 46, 191 - 198 (July-August 1995).

Pour remédier à ces problèmes, il a notamment été proposé d'utiliser des esters de rétinyle, relativement plus stables que le rétinol. Toutefois, la stabilité de ces composés dans des compositions cosmétiques reste insuffisante, en particulier s'agissant des esters du rétinol avec un acide à chaîne grasse.

La Demanderesse a en particulier noté que l'introduction de certains filtres UVA dans des compositions cosmétiques comprenant du rétinol ou l'un de ses esters occasionnait une dégradation du rétinoïde dans le temps, sans que les raisons de cette dégradation puissent être clairement identifiées.

Cela est particulièrement fâcheux s'agissant de compositions destinées à la prévention ou au traitement des signes cutanés du photo-vieillissement, dans la mesure où il est souvent intéressant de cumuler, dans ces compositions, les effets biologiques du rétinoïde, notamment sur la synthèse de collagène, aux effets des filtres solaires absorbant le rayonnement UVA. Ces filtres permettent en effet d'aider les cellules à se défendre contre l'excès de radicaux libres photo-induits et de prévenir la dégradation des fibres de collagène due aux UVA. Ils ont donc des effets anti-âge complémentaires de ceux du rétinoïde.

On comprend donc l'importance qui existe à disposer de compositions, notamment cosmétiques, comprenant à la fois un rétinoïde et un filtre UV, en particulier un filtre UVA, dans lesquelles le rétinoïde ne se trouve pas dégradé par le filtre.

Or, la Demanderesse a découvert avec étonnement qu'une famille donnée de filtres UVA pouvait être introduite dans des compositions comprenant un rétinoïde sans entraîner de dégradation de celui-ci. Ces compositions cosmétiques et/ou dermatologiques peuvent ainsi être stockées pendant plusieurs mois, sans voir se dégrader leur efficacité.

L'invention a donc pour objet une composition comprenant, dans un milieu physiologiquement acceptable, au moins un rétinoïde et au moins une silicone benzotriazole choisie parmi les silicones benzotriazoles répondant à l'une des formules suivantes: 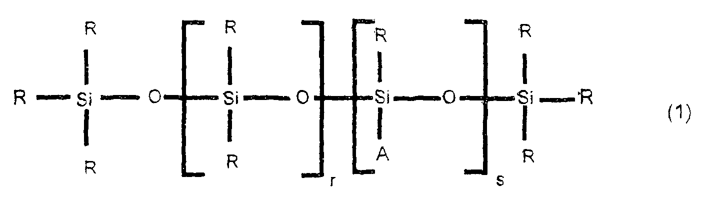 ou 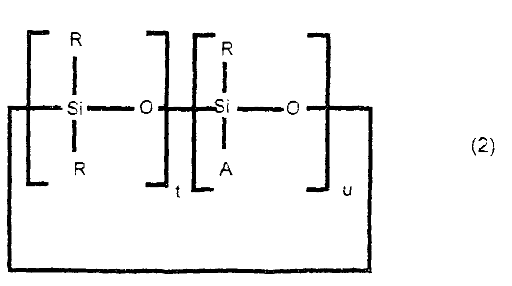 formules (1) et (2) dans lesquelles :
- R, identiques ou différents, sont choisis parmi les radicaux alkyles en C₁-C₁₀, phényle et trifluoro-3,3,3 propyle, au moins 80% en nombre des radicaux R étant méthyle,
- r est un nombre entier compris entre 0 et 50 inclusivement, et s est un nombre entier compris entre 1 et 20 inclusivement,
- u est un nombre entier compris entre 1 et 6 inclusivement, et t est un nombre entier compris entre 0 et 10 inclusivement, étant entendu que t + u est égal ou supérieur à 3,
- et le symbole A désigne un radical monovalent lié directement à un atome de silicium, et qui répond à la formule (3) suivante : 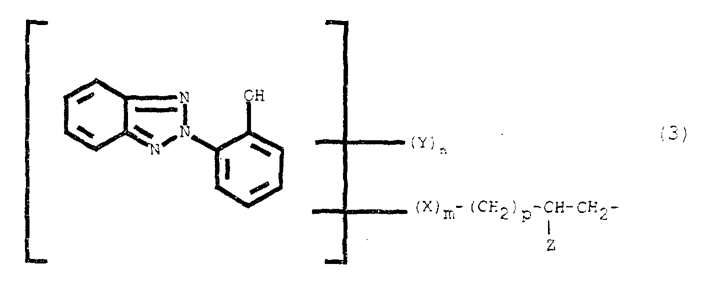 formule (3) dans laquelle :
   - Y, identiques ou différents, sont choisis parmi les radicaux alkyles en C₁-C₈, les halogènes et les radicaux alkoxy en C₁-C₄ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
   - X représente O ou NH,
   - Z représente l'hydrogène ou un radical alkyle en C₁-C₄,
   - n est un nombre entier compris entre 0 et 3 inclusivement,
   - m est 0 ou 1,
   - p représente un nombre entier compris entre 1 et 10, inclusivement.

Comme cela ressort de la formule (3) donnée ci-dessus, l'accrochage du chaînon -(X)ₘ-(CH₂)ₚ-CH(Z)-CH₂- sur le motif benzotriazole, qui assure donc le raccordement dudit motif benzotriazole à l'atome de silicium de la chaîne siliconée, peut, selon la présente invention, se faire dans toutes les positions disponibles offertes par les deux noyaux aromatiques du benzotriazole :

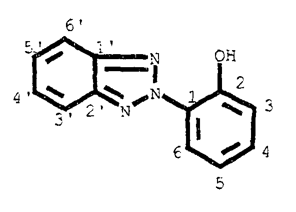

De préférence, cet accrochage se fait en position 3, 4, 5 (noyau aromatique portant la fonction hydroxy) ou 4' (noyau benzénique adjacent le cycle triazolé), et encore plus préférentiellement en position 3, 4 ou 5. Dans une forme préférée de réalisation de l'invention, l'accrochage se fait en position 3.

De même, l'accrochage du motif substituant Y peut se faire dans toutes les autres positions disponibles au sein du benzotriazole. Toutefois, de préférence, cet accrochage se fait en position 3, 4, 4', 5 et/ou 6. Dans une forme préférée de réalisation de l'invention, l'accrochage se fait en position 5.

Dans les formules (1) et (2) ci-dessus, les radicaux alkyle peuvent être linéaires ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Les radicaux alkyle R préférés selon l'invention sont les radicaux méthyle, éthyle, propyle, n-butyle, n-octyle et éthyl-2 hexyle. Encore plus préférentiellement, les radicaux R sont tous des radicaux méthyle.

Parmi les composés de formules (1) et (2) ci-dessus, on préfère mettre en oeuvre ceux répondant à la formule (1), c'est-à-dire des diorganosiloxanes à chaîne courte linéaire.

Parmi les diorganosiloxanes linéaires rentrant dans le cadre de la présente invention, on préfère plus particulièrement les dérivés statistiques ou bien définis à blocs présentant au moins l'une, et encore plus préférentiellement l'ensemble, des caractéristiques suivantes :
- R est alkyle et encore plus préférentiellement est méthyle,
- r est compris entre 0 et 15 inclusivement ; s est compris entre 1 et 5 inclusivement,
- n est non nul, et de préférence égal à 1, et Y est alors choisi parmi méthyle, ter.-butyle ou alcoxy en C₁-C₄,
- Z est hydrogène ou méthyle,
- m=0, ou [m=1 et X=O]
- p est égal à 1.

Une famille de composés convenant particulièrement à l'invention est celle définie par la formule générale suivante : 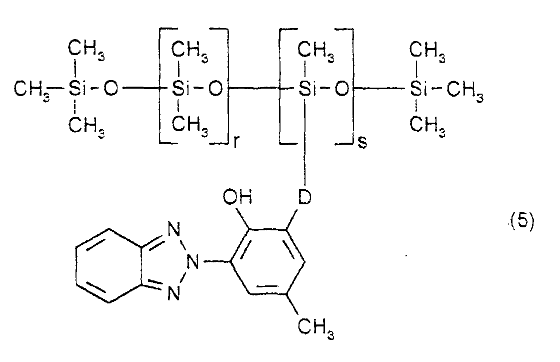 avec
0 ≤ r ≤ 15, de préférence 0 ≤ r ≤ 10
1 ≤ s ≤ 5, de préférence 1 ≤ s ≤ 3
et où D représente le radical divalent : 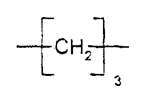 ou

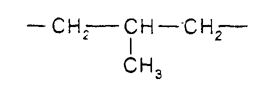

Dans une forme particulièrement préférée de réalisation de l'invention, la silicone benzotriazole répond à la formule générale (6) suivante :

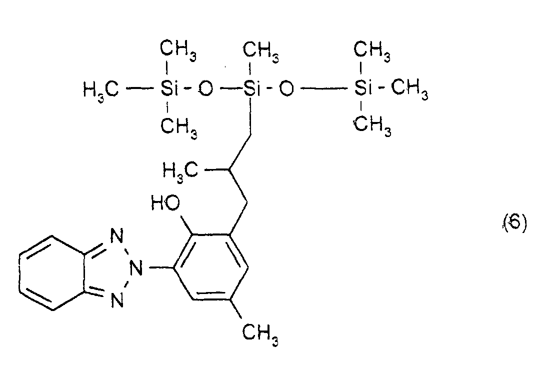

Ces silicones benzotriazoles, ainsi que leur mode de préparation, sont décrits notamment dans la demande FR-A-2 642 968. Elles présentent de bonnes propriétés filtrantes dans une large gamme de longueurs d'ondes allant de 280 nm à 360 nm, ainsi que de bonnes propriétés cosmétiques en raison de leur solubilité aisée dans les corps gras.

Le rétinoïde selon l'invention peut être le rétinol, le rétinal ou un ester de rétinyle tel qu'un ester de rétinol et d'un acide en C₂-C₂₀, tel que le propionate, l'acétate ou le palmitate de rétinyle.

Selon une forme d'exécution préférée de l'invention, le rétinoïde est le rétinol. Par ce terme, on entend tous les isomères du rétinoi, à savoir le rétinol all-trans, le rétinol 13-cis, le rétinol 11-cis, le rétinol 9-cis et le 3,4-didéhydrorétinol. On préfère utiliser le rétinol *all-trans.*

La composition selon l'invention est de préférence destinée à un usage cosmétique ou dermatologique, avantageusement cosmétique. Elle est destinée à une application topique sur la peau et contient donc généralement un milieu physiologiquement acceptable, c'est-à-dire compatible avec la peau.

La composition selon l'invention comprend en général une quantité de rétinoïde efficace pour obtenir l'effet recherché, par exemple comprise entre 0,01 et 0,2% en poids, et de préférence comprise entre 0,01 et 0,15 % en poids, par rapport au poids total de la composition. Elle renferme en outre une quantité de silicone benzotriazole suffisante pour lui conférer le Facteur de Protection Solaire voulu, par exemple de 0,5 à 15% en poids, et de préférence de 0,7 à 5% en poids, de silicone benzotriazole, par rapport au poids total de la composition.
Pour une meilleure protection vis-à-vis des UV, la composition selon l'invention peut, en plus de la silicone benzotriazole précitée, renfermer au moins un autre composé capable de filtrer le rayonnement UVA et/ou au moins un autre composé capable de filtrer le rayonnement UVB et/ou au moins un pigment inorganique éventuellement enrobé.

Comme autre composé capable de filtrer le rayonnement UVA, on peut notamment citer :
(1) les dérivés de benzophénone, par exemple :
   - la 2,4-dihydroxybenzophénone (benzophénone-1) ;
   - la 2,2',4,4'-tétra-hydroxybenzophénone (benzophénone-2) ;
   - la 2-hydroxy-4-méthoxy-benzophénone (benzophénone-3), disponible auprès de la société BASF sous la dénomination commerciale UVINUL M40 ;
   - l'acide 2-hydroxy-4-méthoxy-benzophénone-5-sulfonique (benzophénone-4) ainsi que sa forme sulfonate (benzophénone-5), disponible auprès de la société BASF sous la dénomination commerciale UVINUL MS40 ;
   - la 2,2'-dihydroxy-4,4'-diméthoxy-benzophénone (benzophénone-6) ;
   - la 5-chloro-2-hydroxybenzophénone (benzophénone-7) ;
   - la 2,2'-dihydroxy-4-méthoxy-benzophénone (benzophénone-8) ;
   - le sel disodique du diacide 2,2'-dihydroxy-4,4'-diméthoxy-benzophénone-5,5'-disulfonique (benzophénone-9) ;
   - la 2-hydroxy-4-méthoxy-4'-méthyl-benzophénone (benzophénone-10) ;
   - la benzophénone-11 ;
   - la 2-hydroxy-4-(octyloxy)benzophénone (benzophénone-12), les benzophénones 3 et 5 étant préférées ;
(2) les dérivés de triazine, et en particulier la 2,4-bis [4-(2-éthyl-hexyloxy)-2-hydroxyphényl]-6-(4-méthoxy-phényl)-1,3,5-triazine disponible auprès de la société CIBA GEIGY sous la dénomination commerciale TINOSORB S et le 2,2'-méthylènebis-[6-(2H benzotriazol-2-yl)4-(1,1,3,3-tétraméthylbutyl)phénol] disponible auprès de la société CIBA GEIGY sous la dénomination commerciale TINOSORB M ;
(3) les filtres actifs dans l'UV-A de formule (I) suivante : 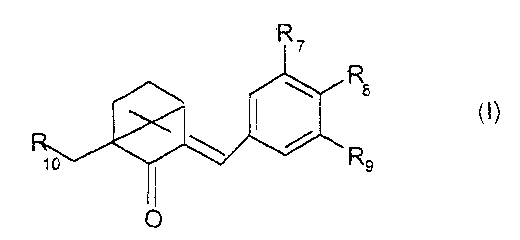 dans laquelle :
   - R₇ et R₉, identiques ou différents, désignent un hydrogène, un halogène, un radical OH, un radical alkyle, saturé ou non, linéaire ou ramifié en C₁-C₁₀ ; un radical alcoxy, saturé ou non, linéaire ou ramifié en C₁-C₁₀ ou un groupe HSO₃ ;
   - R₁₀ désigne hydrogène ou HSO₃ ;
   - R₈ désigne hydroxy ; ou un groupe OR₁₁ où R₁₁ désigne un radical alkyle, saturé ou insaturé, linéaire ou ramifié en C₁-C₁₀ ; ou bien un groupe de structure suivante : 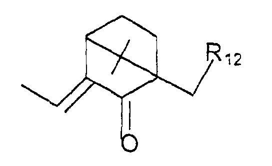
   dans laquelle R₁₂ désigne hydrogène ou HSO₃;
   ou bien un groupe de formule suivante : 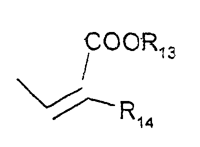 ou bien un groupe de formule suivante : 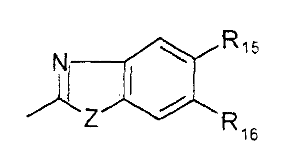 dans lesquelles :
   - Z désigne un atome d'oxygène ou un radical -NH- ;
   - R₁₃, R₁₄, R₁₅ et R₁₆, identiques ou différents, désignent un hydrogène, un halogène, un radical OH, un radical alkyle, saturé ou non, linéaire ou ramifié en C₁-C₁₀ ; un radical alcoxy, saturé ou non, linéaire ou ramifié en C₁-C₁₀ ou un groupe HSO₃,
   en particulier l'acide benzène-1,4-di(3-méthylidènecampho-10-sulfonique) ou l'un de ses sels de métal alcalin ou alcalino-terreux, d'ammonium ou avec une base organique, qui répond à la formule suivante : 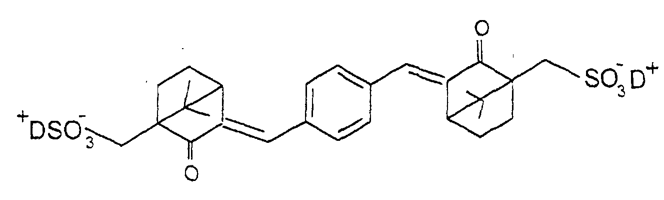 dans laquelle D désigne un atome d'hydrogène, un métal alcalin ou un radical NH(R₂₅)₃⁺ dans lequel les radicaux R₂₅, qui peuvent être identiques ou différents, désignent un atome d'hydrogène, un radical alkyle ou hydroxyalkyle en C₁-C₄ ou encore un groupement Mⁿ⁺/n, Mⁿ⁺ désignant un cation métallique polyvalent dans lequel n est est égal à 2 ou 3 ou 4, Mⁿ⁺ désignant de préférence un cation métallique choisi parmi Ca²⁺, Zn²⁺, Mg²⁺, Ba²⁺, Al³⁺ et Zr⁴⁺.
(4) leurs mélanges.

Comme autre composé capable de filtrer le rayonnement UVB, on peut notamment citer :
(1) les dérivés de l'acide salicylique, en particulier le salicylate d'homomenthyle et le salicylate d'octyle ;
(2) les dérivés de l'acide cinnamique, en particulier le p-méthoxycinnamate de 2-éthylhexyle, disponible auprès de la société GIVAUDAN sous la dénomination commerciale Parsol MCX ;
(3) les dérivés de β,β'-diphénylacrylate liquides, en particulier l'α-cyano-β,β' diphénylacrylate de 2-éthylhexyle, ou octocrylène, disponible auprès de la société BASF sous la dénomination commerciale UVINUL N539 ;
(4) les dérivés de l'acide p-aminobenzoïque ;
(5) le 4-méthyl benzylidène camphre disponible auprès de la société MERCK, sous la dénomination commerciale EUSOLEX 6300 ;
(6) l'acide 2-phénylbenzimidazole 5-sulfonique vendu sous la dénomination commerciale « EUSOLEX 232 » par la société MERCK
(7) les dérivés de 1,3,5-triazine, en particulier :
   - la 2,4,6-tris[4-(éthylhexyloxycarbonyl)anilino]-1,3,5-triazine, disponible auprès de la société BASF sous la dénomination commerciale UVINUL T150, et
   - le composé répondant à la formule suivante : 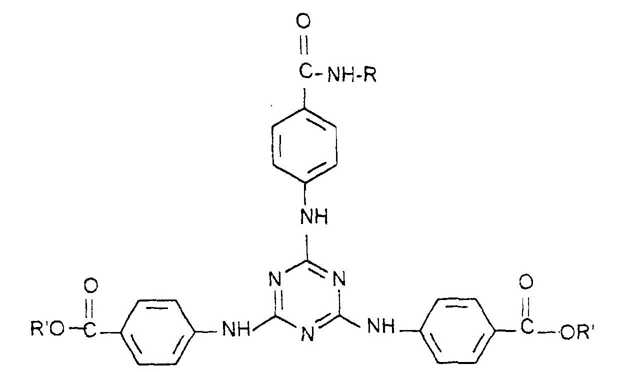 dans laquelle R' désigne un radical éthyl-2 hexyle et R désigne un radical tert- butyle, disponible auprès de la société SIGMA 3V sous la dénomination commerciale UVASORB HEB ;
(8) leurs mélanges.

Les pigments inorganiques éventuellement enrobés utilisables dans la composition selon l'invention sont en particulier les nanopigments (taille moyenne des particules primaires : généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

La composition selon l'invention peut se présenter sous toutes les formes galéniques normalement utilisées dans les domaines cosmétique et dermatologique, et elle peut être notamment sous forme d'une solution aqueuse éventuellement gélifiée, d'une dispersion du type lotion éventuellement biphasée, d'une émulsion obtenue par dispersion d'une phase grasse dans une phase aqueuse (H/E) ou inversement (E/H), d'une émulsion triple (E/H/E ou H/E/H) ou d'une dispersion vésiculaire de type ionique et/ou non ionique. Ces compositions sont préparées selon les méthodes usuelles. De préférence, la composition selon l'invention est sous la forme d'une émulsion huile-dans-eau.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol peut également se présenter sous forme solide, en particulier sous forme de stick pour les lèvres. Elle peut être utilisée comme produit de soin et/ou comme produit de maquillage pour la peau.

De façon connue, la composition de l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse, ou dans les vésicules lipidiques. En tout état de cause, ces adjuvants, ainsi que leurs proportions, seront choisis de manière à ne pas nuire aux propriétés recherchées de l'association selon l'invention.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-100, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et ies polyéthylènes.

La présente invention concerne également les utilisations cosmétiques de la composition décrite précédemment, notamment pour prévenir ou traiter les signes du vieillissement cutané intrinsèque ou photo-induit.

Elle concerne aussi l'utilisation de la composition décrite précédemment pour fabriquer une préparation destinée à prévenir ou traiter les dommages cutanés dus aux UV.

L'invention sera mieux comprise, et ses avantages ressortiront mieux, à la lumière des exemples suivants, qui sont donnés à titre illustratif.

### EXEMPLES

### Exemple 1 : Composition cosmétique

| *Phase A* | |
|---|---|
| Stéarate de glycéryle et stéarate de PEG-100 | 2,1 % |
| Polysorbate 60 | 0,9 % |
| Alcool cétylique | 2,6 % |
| Polyisobutène hydrogéné | 12 % |
| Hexyldécanol | 8 % |
| BHT | 0,1 % |
| Conservateur | 0,15% |
| Silicone benzotriazole de formule (6) | 3 % |

| *Phase B* | |
|---|---|
| Eau | qsp 100 % |
| Glycérine | 3 % |
| Conservateur | 0,55% |
| Sel pentasodique d'acide éthylenediamine tétraméthylène phosphonique | 0,07% |

| *Phase C* | |
|---|---|
| Gomme de xanthane | 0,1 % |
| Carbomer | 0,4 % |

| *Phase D* | |
|---|---|
| Eau | 5 % |
| Triéthanolamine | 0,38% |

| *Phase E* | |
|---|---|
| Rétinol | 0,1 % |

La composition ci-dessus peut être préparée de la manière suivante.

Les phases A et B sont chauffées séparément sous agitation à 75°C jusqu'à parfaite solubilisation. La phase D est préparée séparément sous agitation à température ambiante. La phase A est ensuite transvasée dans la phase B sous agitation à 75°C pendant 5-10 min, puis l'ensemble est refroidi à 50°C. Les constituants de la phase C sont ensuite introduits dans le mélange des phases A et B sous agitation à 50°C, et après homogénéisation complète, on ajoute successivement la phase D sous agitation. Le mélange est refroidi à température ambiante. Après réalisation d'un inertage à l'azote, la phase E est introduite en cuve sous agitation.

Cette composition peut être utilisée quotidiennement comme crème de jour pour prévenir et lutter contre les rides et raffermir la peau.

### Exemple 2 : Mise en évidence de la stabilisation du rétinol

La stabilité du rétinol a été évaluée dans les trois compositions A à C ci-après :
Composition A : Composition de l'Exemple 1 sans la silicone benzotriazole
Composition B : Composition de l'Exemple 1
Composition C : Composition de l'Exemple 1 dans laquelle la silicone benzotriazole est remplacée par 3% de 4-tert-butyl-4'-méthoxydibenzoylméthane (commercialisé sous la dénomination Parsol 1789 par la société GIVAUDAN) dans la phase A.

Les quantités pondérales de rétinol respectivement présentes dans les compositions A à C ci-dessus ont été déterminées, d'une part, à température ambiante, immédiatement après préparation (To) de ces compositions, et, d'autre part, après deux mois de conservation à 45°C (T2m).

Ces mesures ont été effectuées par Chromatographie Liquide Haute Pression, en utilisant comme étalon une solution de rétinol à 16 µg/ml dans le THF, préparée à partir d'un échantillon de Rétinol 10S commercialisé par la société BASF sous la forme d'une solution huileuse à 10% de rétinol.

Les résultats obtenus sont les suivants :

| | To | T2m |
|---|---|---|
| Composition A | 0,108 % | 0,102 % |
| Composition B | 0,106 % | 0,103 % |
| Composition C | 0,105 % | 0,061 % |

Compte tenu de la précision de la méthode de dosage utilisée, on peut en déduire que la composition B comprenant, comme filtre UVA, la silicone benzotriazole, est aussi stable que la composition A ne contenant pas de filtre UV et nettement plus stable que la composition C comprenant un autre filtre UVA.

## Revendications

1. Composition comprenant, dans un milieu physiologiquement acceptable, au moins un rétinoïde et au moins une silicone benzotriazole choisie parmi les silicones benzotriazoles répondant à l'une des formules suivantes: 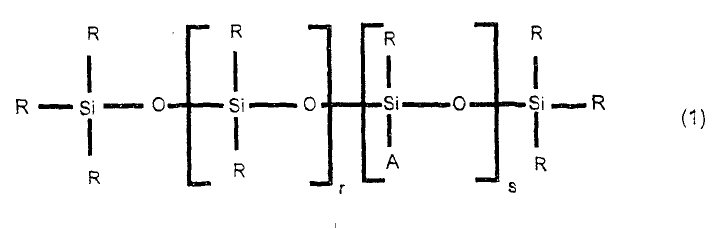 ou 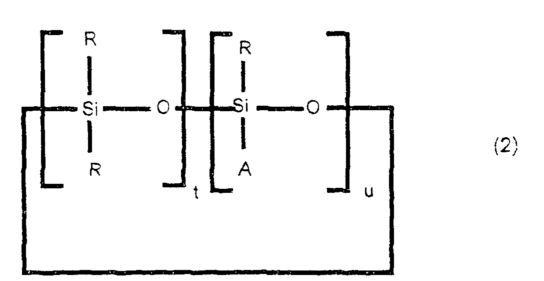 formules (1) et (2) dans lesquelles :
- R, identiques ou différents, sont choisis parmi les radicaux alkyles en C₁-C₁₀, phényle et trifluoro-3,3,3 propyle, au moins 80% en nombre des radicaux R étant méthyle,
- r est un nombre entier compris entre 0 et 50 inclusivement, et s est un nombre entier compris entre 1 et 20 inclusivement,
- u est un nombre entier compris entre 1 et 6 inclusivement, et t est un nombre entier compris entre 0 et 10 inclusivement, étant entendu que t + u est égal ou supérieur à 3,
- et le symbole A désigne un radical monovalent lié directement à un atome de silicium, et qui répond à la formule (3) suivante : 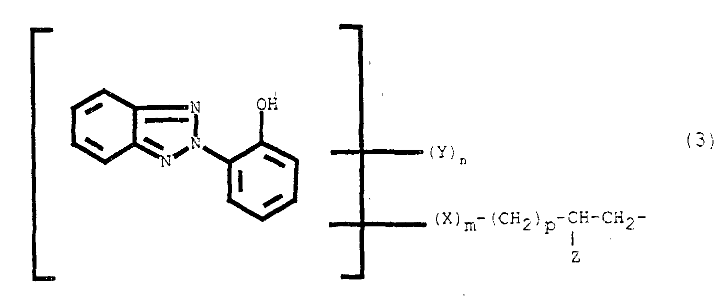 formule (3) dans laquelle :
- Y, identiques ou différents, sont choisis parmi les radicaux alkyles en C₁-C₈, les halogènes et les radicaux alkoxy en C₁-C₄ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en C₁-C₄,
- n est un nombre entier compris entre 0 et 3 inclusivement,
- m est 0 ou 1,
- p représente un nombre entier compris entre 1 et 10, inclusivement.

2. Composition selon la revendication 1, **caractérisée par le fait que** la silicone benzotriazole est choisie parmi les composés de formule générale (1) présentant au moins l'une des caractéristiques suivantes :
- R est alkyle, et de préférence méthyle,
- r est compris entre 0 et 15 inclusivement ; s est compris entre 1 et 5 inclusivement,
- n est non nul, ou égal à 1, et Y est alors choisi parmi méthyle, ter.-butyle ou alcoxy en C₁-C₄,
- Z est hydrogène ou méthyle,
- m=0, ou [m=1 et X=O]
- p est égal à 1.

3. Composition selon la revendication 2, **caractérisée par le fait que** la silicone benzotriazole est choisie parmi les composés de formule générale (5) : 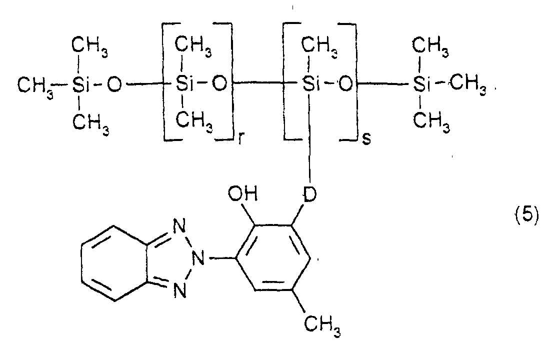 où
0 ≤ r ≤ 15
1 ≤ s ≤ 5
et où D représente le radical divalent : 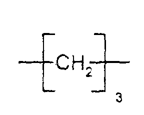 ou

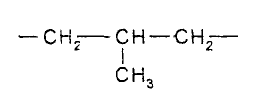

4. Composition selon la revendication 3, **caractérisée par le fait que** la silicone benzotriazole répond à la formule générale (6) :

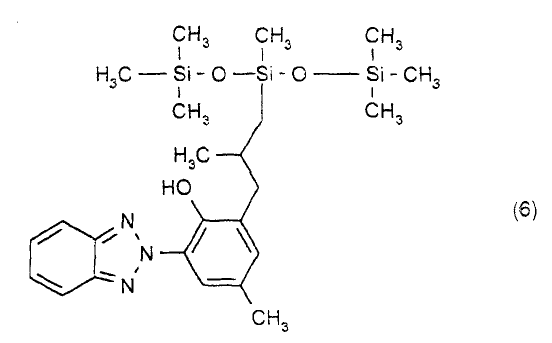

5. Composition selon l'une quelconque des revendications 1 à 4, **caractérisée en ce que** ledit rétinoïde est choisi parmi le rétinol, le rétinal et les esters de rétinyle.

6. Composition selon la revendication 5, **caractérisée en ce que** lesdits esters de rétinyle sont choisis parmi les esters du rétinol avec un acide en C₂-C₂₀, tel que le propionate, l'acétate ou le palmitate de rétinyle.

7. Composition selon la revendication 5, **caractérisée en ce que** le rétinoïde est le rétinol.

8. Composition selon l'une quelconque des revendications 1 à 7, **caractérisée en ce qu'**elle comprend de 0,01 à 0,15% en poids de rétinoïde, par rapport au poids total de la composition.

9. Composition selon l'une quelconque des revendications 1 à 8, **caractérisée en ce qu'**elle renferme de 0,7 à 5% en poids de silicone benzotriazole, par rapport au poids total de la composition.

10. Composition selon l'une quelconque des revendications 1 à 9, **caractérisée en ce qu'**elle est sous la forme d'une émulsion huile-dans-eau.

11. Composition selon l'une quelconque des revendications 1 à 10, **caractérisée en ce qu'**elle renferme en outre au moins un autre composé capable de filtrer le rayonnement UVA et/ou au moins un autre composé capable de filtrer le rayonnement UVB et/ou au moins un pigment inorganique éventuellement enrobé.

12. Composition selon la revendication 11, **caractérisée en ce que** ledit autre composé capable de filtrer le rayonnement UVA est choisi parmi :
(1) les dérivés de benzophénone,
(2) les dérivés de triazine,
(3) l'acide benzène 1,4-di(3-méthylidène-10-camphosulfonique) ou l'un de ses sels de métal alcalin ou alcalino-terreux, d'ammonium ou avec une base organique, et
(4) leurs mélanges.

13. Composition selon la revendication 11, **caractérisée en ce que** ledit autre composé capable de filtrer le rayonnement UVA est choisi parmi :
(1) la 2-hydroxy-4-méthoxy-benzophénone (benzophénone-3) et l'acide 2-hydroxy-4-méthoxy-benzophénone-5-sulfonique,
(2) la 2,4-bis [4-(2-éthyl-hexyloxy)-2-hydroxyphényl]-6-(4-méthoxy-phényl)-1,3,5-triazine et le 2,2'-méthylènebis-[6-(2H benzotriazol-2-yl)4-(1,1,3,3-tétraméthylbutyl)phénol], et
(3) l'acide benzène 1,4-di(3-méthylidène-10-camphosulfonique) ou l'un de ses sels de métal alcalin ou alcalino-terreux, d'ammonium ou avec une base organique.

14. Composition selon l'une des revendications 11 à 13, **caractérisée en ce que** ledit autre composé capable de filtrer le rayonnement UVB est choisi parmi :
(1) les dérivés de l'acide salicylique,
(2) les dérivés de l'acide cinnamique,
(3) les dérivés de β,β'-diphénylacrylate liquides,
(4) les dérivés de l'acide p-aminobenzoïque ;
(5) le 4-méthyl benzylidène camphre ;
(6) l'acide 2-phénylbenzimidazole 5-sulfonique ;
(7) les dérivés de 1,3,5-triazine,
(8) leurs mélanges.

15. Composition selon l'une des revendications 11 à 14, **caractérisée en ce que** ledit autre composé capable de filtrer le rayonnement UVB est choisi parmi :
(1) le salicylate d'homomenthyle et le salicylate d'octyle,
(2) le p-méthoxycinnamate de 2-éthylhexyle,
(3) l'α-cyano-α,β' diphénylacrylate de 2-éthylhexyle, ou octocrylène ,
(4) le 4-méthyl benzylidène camphre,
(5) l'acide 2-phénylbenzimidazole 5-sulfonique,
(6) la 2,4,6-tris[4-(éthylhexyloxycarbonyl)anilino]-1,3,5-triazine, et
(7) le composé répondant à la formule suivante : 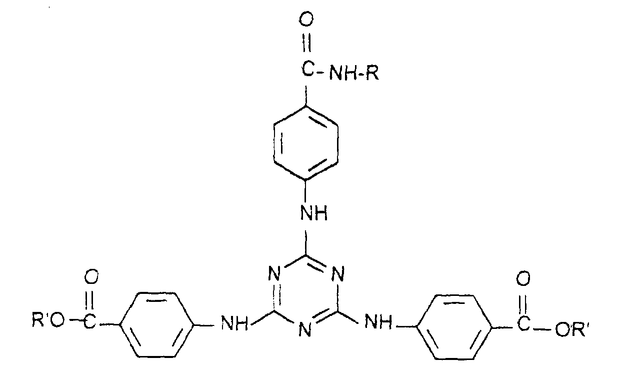 dans laquelle R' désigne un radical éthyl-2 hexyle et R désigne un radical tert- butyle

16. Composition selon la revendication 11, **caractérisée en ce que** ledit pigment inorganique est choisi parmi les nanopigments d'oxyde de titane, de fer, de zinc, de zirconium ou de cérium éventuellement enrobés d'alumine et/ou de stéarate d'aluminium.

17. Utilisation cosmétique de la composition selon l'une quelconque des revendications 1 à 16 pour prévenir ou traiter les signes du vieillissement cutané intrinsèque ou photo-induit.

18. Utilisation de la composition selon l'une quelconque des revendications 1 à 16 pour fabriquer une préparation destinée à prévenir ou traiter les dommages cutanés dus aux UV.

## Claims

1. Composition comprising, in a physiologically acceptable medium, at least one retinoid and at least one benzotriazole silicone chosen from the benzotriazole silicones corresponding to one of the following formulae: 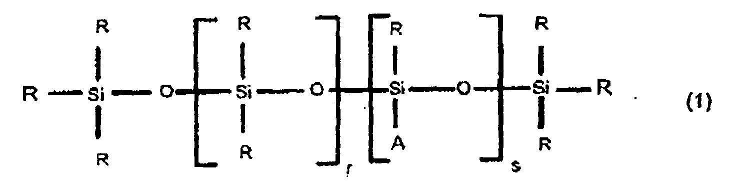 or 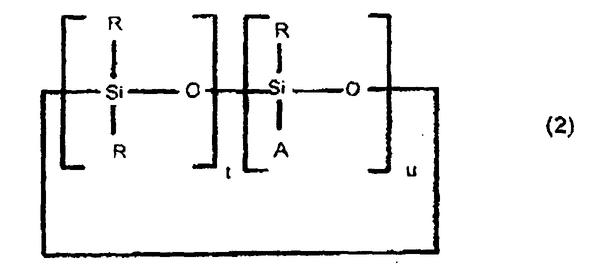 in which formulae (1) and (2):
- R, which are identical or different, are chosen from C₁-C₁₀ alkyl, phenyl and 3,3,3-trifluoropropyl radicals, at least 80% by number of the R radicals being methyl,
- r is an integer of between 0 and 50 inclusive and s is an integer of between 1 and 20 inclusive,
- u is an integer of between 1 and 6 inclusive and t is an integer of between 0 and 10 inclusive, it being understood that t + u is equal to or greater than 3,
- and the symbol A denotes a monovalent radical bonded directly to a silicon atom which corresponds to the following formula (3): 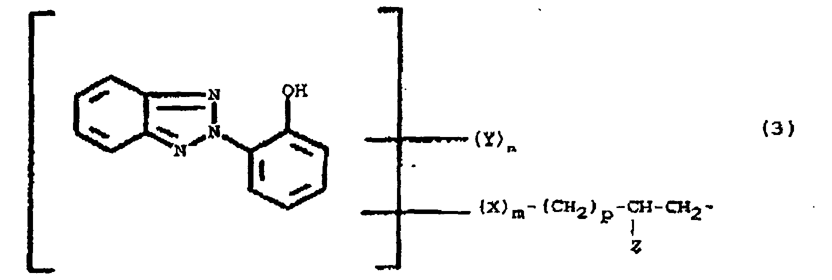 in which formula (3):
- Y, which are identical or different, are chosen from C₁-C₈ alkyl radicals, halogens and C₁-C₄ alkoxy radicals, it being understood that, in the latter case, two adjacent Y radicals of the same aromatic nucleus can together form an alkylidenedioxy group in which the alkylidene group comprises from 1 to 2 carbon atoms,
- X represents O or NH,
- Z represents hydrogen or a C₁-C₄ alkyl radical,
- n is an integer of between 0 and 3 inclusive,
- m is 0 or 1,
- p represents an integer of between 1 and 10 inclusive.

2. Composition according to Claim 1, **characterized in that** the benzotriazole silicone is chosen from the compounds of general formula (1) exhibiting at least one of the following characteristics:
- R is alkyl and preferably methyl,
- r is between 0 and 15 inclusive; s is between 1 and 5 inclusive,
- n is non-zero, equal to 1 and Y is then chosen from methyl, tert-butyl or C₁-C₄ alkoxy,
- Z is hydrogen or methyl,
- m = 0 or [m = 1 and X = O],
- p is equal to 1.

3. Composition according to Claim 2, **characterized in that** the benzotriazole silicone is chosen from the compounds of general formula (5): 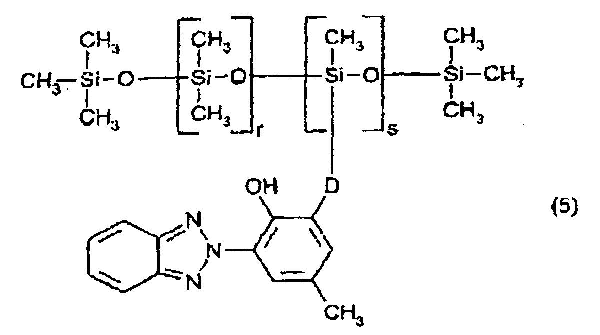 with 0 ≤ r ≤ 15,
1 **≤** s ≤ 5,
and where D represents the divalent radical: 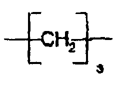 or

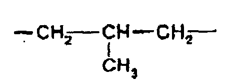

4. composition according to Claim 3, **characterized in that** the benzotriazole silicone corresponds to the general formula (6):

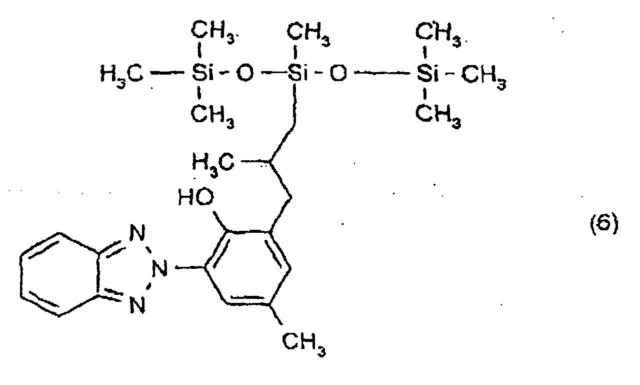

5. Composition according to any one of Claims 1 to 4, **characterized in that** the said retinoid is chosen from retinol, retinal and retinyl esters.

6. Composition according to Claim 5, **characterized in that** the said retinyl esters are chosen from esters of retinol with a C₂-C₂₀ acid, such as retinyl propionate, acetate or palmitate.

7. Composition according to Claim 5, **characterized in that** the retinoid is retinol.

8. Composition according to any one of Claims 1 to 7, **characterized in that** it comprises from 0.01 to 0.15% by weight of retinoid, with respect to the total weight of the composition.

9. Composition according to any one of Claims 1 to 8, **characterized in that** it includes from 0.7 to 5% by weight of benzotriazole silicone, with respect to the total weight of the composition.

10. Composition according to any one of Claims 1 to 9, **characterized in that** it is in the form of an oil-in-water emulsion.

11. Composition according to any one of Claims 1 to 10, **characterized in that** it additionally includes at least one other compound capable of screening out UVA radiation and/or at least one other compound capable of screening out UVB radiation and/or at least one optionally coated inorganic pigment.

12. Composition according to Claim 11, **characterized in that** the said other compound capable of screening out UVA radiation is chosen from:
(1) benzophenone derivatives,
(2) triazine derivatives,
(3) benzene-1,4-di(3-methylidene-10-camphorsulphonic acid) or one of its alkali metal, alkaline earth metal or ammonium salts or one of its salts with an organic base, and
(4) their mixtures.

13. Composition according to Claim 11, **characterized in that** the said other compound capable of screening out UVA radiation is chosen from:
(1) 2-hydroxy-4-methoxybenzophenone (benzophenone-3) and 2-hydroxy-4-methoxybenzophenone-5-sulphonic acid,
(2) 2,4-bis[4-(2-ethylhexyloxy)-2-hydroxyphenyl]-6-(4-methoxyphenyl)-1,3,5-triazine and 2,2'-methylene-bis[6-(2H-benzotriazol-2-yl)-4-(1,1,3,3-tetramethylbutyl)phenol], and
(3) benzene-1,4-di(3-methylidene-10-camphorsulphonic acid) or one of its alkali metal, alkaline earth metal or ammonium salts or one of its salts with an organic base.

14. Composition according to one of Claims 11 to 13, **characterized in that** the said other compound capable of screening out UVB radiation is chosen from:
(1) salicylic acid derivatives,
(2) cinnamic acid derivatives,
(3) liquid β,β'-diphenylacrylate derivatives,
(4) p-aminobenzoic acid derivatives;
(5) 4-methylbenzylidenecamphor;
(6) 2-phenylbenzimidazole-5-sulphonic acid;
(7) 1,3,5-triazine derivatives,
(8) their mixtures.

15. Composition according to one of Claims 11 to 14, **characterized in that** the said other compound capable of screening out UVB radiation is chosen from:
(1) homomenthyl salicylate and octyl salicylate;
(2) 2-ethylhexyl p-methoxycinnamate,
(3) 2-ethylhexyl α-cyano-β,β'-diphenylacrylate, or octocrylene,
(4) 4-methylbenzylidenecamphor,
(5) 2-phenylbenzimidazole-5-sulphonic acid,
(6) 2,4,6-tris[4-(ethylhexyloxycarbonyl)anilino]-1,3,5-triazine, and
(7) the compound corresponding to the following formula: 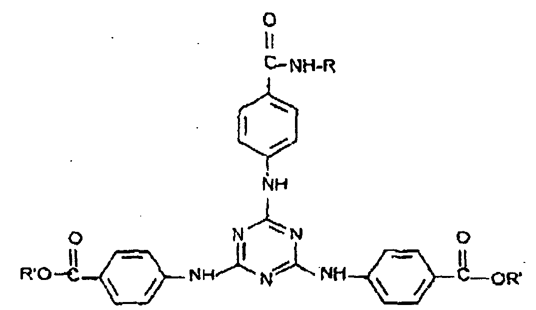 in which R' denotes a 2-ethylhexyl radical and R denotes a tert-butyl radical.

16. Composition according to Claim 11,
**characterized in that** the said inorganic pigment is chosen from titanium oxide, iron oxide, zinc oxide, zirconium oxide or cerium oxide nanopigments optionally coated with alumina and/or with aluminium stearate.

17. Cosmetic use of the composition according to any one of Claims 1 to 16 for preventing or treating signs of intrinsic or photoinduced cutaneous ageing.

18. Use of the composition according to any one of Claims 1 to 16 for manufacturing a preparation intended to prevent or treat cutaneous damage due to UV radiation.

## Patentansprüche

1. Zusammensetzung, die in einem physiologisch verträglichen Medium mindestens ein Retinoid und mindestens ein Benzotriazolsilicon enthält, das einer der folgenden Formeln entspricht: 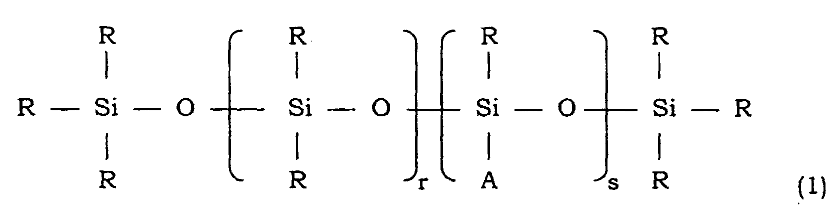 oder 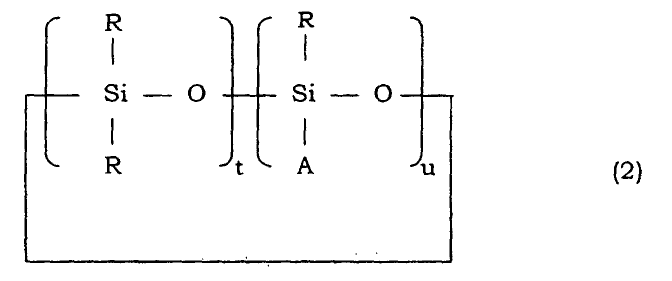 wobei in den Formeln (1) und (2):
· die Gruppen R, die identisch oder voneinander verschieden sind, unter den C₁₋₁₀-Alkylgruppen, Phenyl und 3,3,3-Trifluorpropyl ausgewählt sind, wobei mindestens 80 % der Anzahl der Gruppen R Methyl bedeutet,
· r 0 oder eine ganze Zahl im Bereich von 1 bis 50 und s 0 oder eine ganze Zahl im Bereich von 1 bis 20 bedeutet,
· u eine ganze Zahl im Bereich von 1 bis 6 und t 0 oder eine ganze Zahl im Bereich von 1 bis 10 ist, mit der Maßgabe, dass t + u 3 bedeutet oder darüber liegt, und
· die Gruppe A eine einwertige Gruppe ist, die direkt an ein Siliciumatom gebunden ist und der folgenden Formel (3) entspricht: 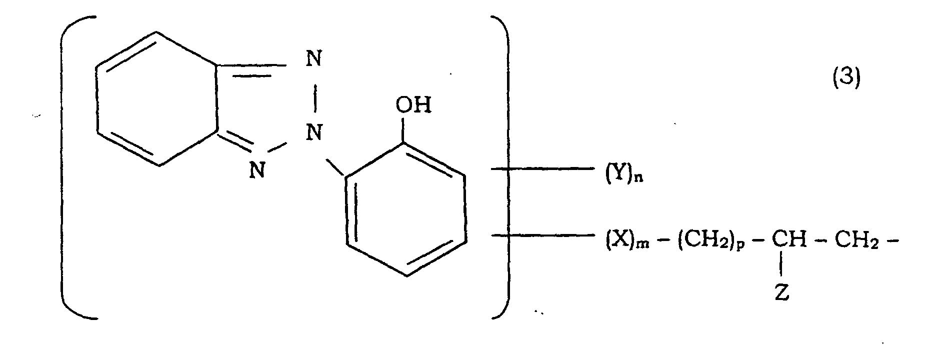 wobei in der Formel (3):
· die Gruppen Y, die identisch oder voneinander verschieden
· sind, unter den C₁₋₈-Alkylgruppen, Halogenen und C₁₋₄-Alkoxygruppen ausgewählt sind, wobei im letzten Fall zwei an dem gleichen aromatischen Ring aneinander angrenzende Gruppen Y auch gemeinsam eine Alkylidendioxygruppe bilden können, worin die Alkylidengruppe 1 bis 2 Kohlenstoffatome aufweist,
· X O oder NH bedeutet,
· Z Wasserstoff oder eine C₁₋₄-Alkylgruppe bedeutet,
· n 0 oder eine ganze Zahl im Bereich von 1 bis 3 ist,
· m 0 oder 1 ist, und
· p eine ganze Zahl im Bereich von 1 bis 10 bedeutet.

2. Zusammensetzung nach Anspruch 1, **dadurch gekennzeichnet, dass** das Benzotriazolsilicon unter den Verbindungen der Formel (1) ausgewählt ist, die mindestens eine der folgenden Eigenschaften aufweisen:
- R bedeutet Alkyl und noch bevorzugter Methyl,
- r liegt im Bereich von 0 bis 15; s liegt im Bereich von 1 bis 5, wobei die Bereichsgrenzen eingeschlossen sind,
- n ist nicht Null und bedeutet 1, und Y ist unter Methyl, t-Butyl oder C₁₋₄-Alkoxy ausgewählt,
- Z bedeutet Wasserstoff oder Methyl,
- m = 0, oder [m = 1 und X = O], und
- p bedeutet 1.

3. Zusammensetzung nach Anspruch 2, **dadurch gekennzeichnet, dass** das Benzotriazolsilicon unter den Verbindungen der folgenden allgemeinen Formel (5) ausgewählt ist: 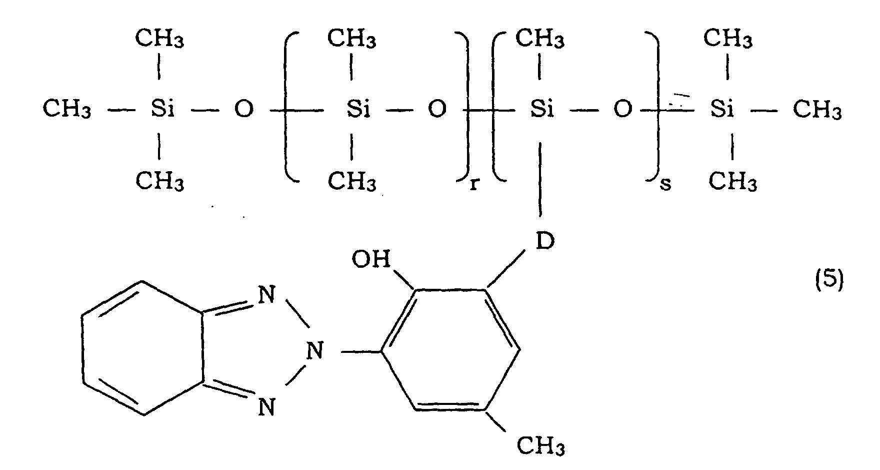 worin bedeuten:
0 ≤ r ≤ 5,
1 ≤ s ≤ 5,
und D die zweiwertige Gruppe: 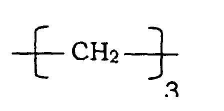 oder

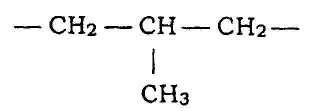

4. Zusammensetzung nach Anspruch 3, **dadurch gekennzeichnet, dass** das Benzotriazolsilicon der allgemeinen Formel (6) entspricht:

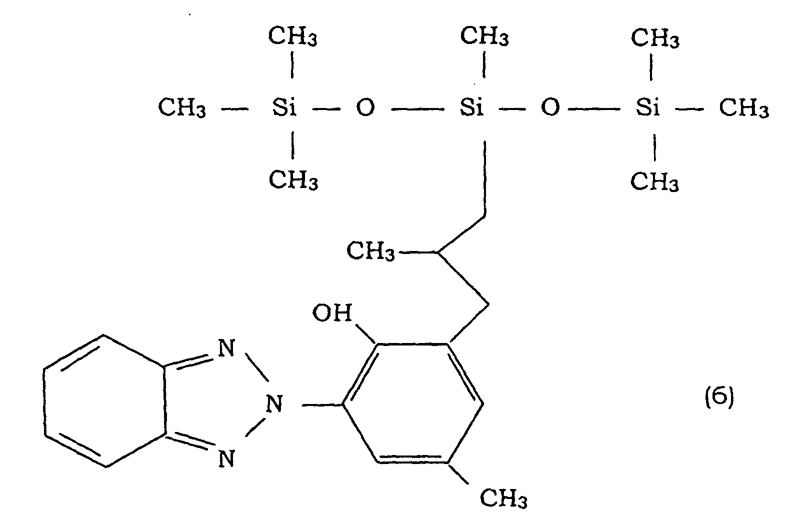

5. Zusammensetzung nach einem der Ansprüche 1 bis 4, **dadurch gekennzeichnet, dass** das Retinoid unter Retinol, Retinal und Retinylestern ausgewählt ist.

6. Zusammensetzung nach Anspruch 5, **dadurch gekennzeichnet, dass** die Retinylester unter den Estern von Retinol mit einer C₂-₂₀-Säure, wie Retinylpropionat, Retinylacetat und Retinylpalmitat ausgewählt ist.

7. Zusammensetzung nach Anspruch 5, **dadurch gekennzeichnet, dass** das Retinoid das Retinol ist.

8. Zusammensetzung nach einem der Ansprüche 1 bis 7, **dadurch gekennzeichnet, dass** sie 0,01 bis 0,15 Gew.-% Retinoid, bezogen auf das Gesamtgewicht der Zusammensetzung, enthält.

9. Zusammensetzung nach einem der Ansprüche 1 bis 8, **dadurch gekennzeichnet, dass** sie 0,7 bis 5 Gew.-% Benzotriazilsilicon, bezogen auf das Gesamtgewicht der Zusammensetzung, enthält.

10. Zusammensetzung nach einem der Ansprüche 1 bis 9, **dadurch gekennzeichnet, dass** sie als Öl-in-Wasser-Emulsion vorliegt.

11. Zusammensetzung nach einem der Ansprüche 1 bis 10, **dadurch gekennzeichnet, dass** sie ferner mindestens eine weitere Verbindung, die die UV-A-Strahlung ausfiltern kann, und/oder mindestens eine Verbindung, die die UV-B-Strahlung ausfiltern kann, und/oder mindestens ein gegebenenfalls umhülltes, organisches Pigment enthält.

12. Zusammensetzung nach Anspruch 11, **dadurch gekennzeichnet, dass** die Verbindung, die die UV-A-Strahlung ausfiltern kann, unter den folgenden Verbindungen ausgewählt ist:
(1) Benzophenonderivaten;
(2) Triazinderivaten;
(3) Benzol-1,4-di(3-methyliden-10-camphersulfonsäure) oder einem ihrer Salze mit Alkalimetallen, Erdalkalimetallen, Ammonium oder einer organischen Base; und
(4) deren Gemischen.

13. Zusammensetzung nach Anspruch 11, **dadurch gekennzeichnet, dass** die weitere Verbindung, die die UV-A-Strahlung ausfiltern kann, unter den folgenden Verbindungen ausgewählt ist:
(1) 2-Hydroxy-4-methoxy-benzophenon (Benzophenon-3) und 2-Hydroxy-4-methoxy-benzophenon-5-sulfonsäure;
(2) 2,4-Bis[4-(2-ethyl-hexyloxy)-2-hydroxyphenyl]-6-(4-methoxyphenyl)-1,3,5-triazin und 2,2'-Methylen-bis[6-(2H-benzotriazol-2-yl)-4-(1,1,3,3-tetramethylbutyl)-phenol]; und
(3) Benzol-1,4-di(3-methyliden-10-camphersulfonsäure) oder einem ihrer Salze mit Alkalimetallen, Erdalkalimetallen, Ammonium oder einer organischen Base.

14. Zusammensetzung nach einem der Ansprüche 11 bis 13, **dadurch gekennzeichnet, dass** die weitere Verbindung, die die UV-B-Strahlung ausfiltern kann, unter den folgenden Verbindungen ausgewählt ist:
(1) Salicylsäurederivaten;
(2) Zimtsäurederivaten;
(3) flüssigen β,β'-Diphenylacrylatderivaten
(4) p-Aminobenzoesäurederivaten;
(5) 4-Methylbenzylidencampher;
(6) 2-Phenylbenzimidazol-5-sulfonsäure;
(7) 1,3,5-Triazinderivaten; und
(8) deren Gemischen.

15. Zusammensetzung nach einem der Ansprüche 11 bis 14, **dadurch gekennzeichnet, dass** die weitere Verbindung, die die UV-B-Strahlung ausfiltern kann, unter den folgenden Verbindungen ausgewählt ist:
(1) Homomenthylsalicylat und Octylsalicylat;
(2) 2-Ethylhexyl-p-methoxycinnamat;
(3) 2-Ethylhexyl-α-Cyano-β,β'-diphenylacrylat oder Octocrylen;
(4) 4-Methylbenzylidencampher;
(5) 2-Phenylbenzimidazol-5-sulfonsäure;
(6) 2,4,6-Tris[4-(ethylhexyloxycarbonyl)anilino]-1,3,5,-triazin;
(7) der Verbindung der folgenden Formel: 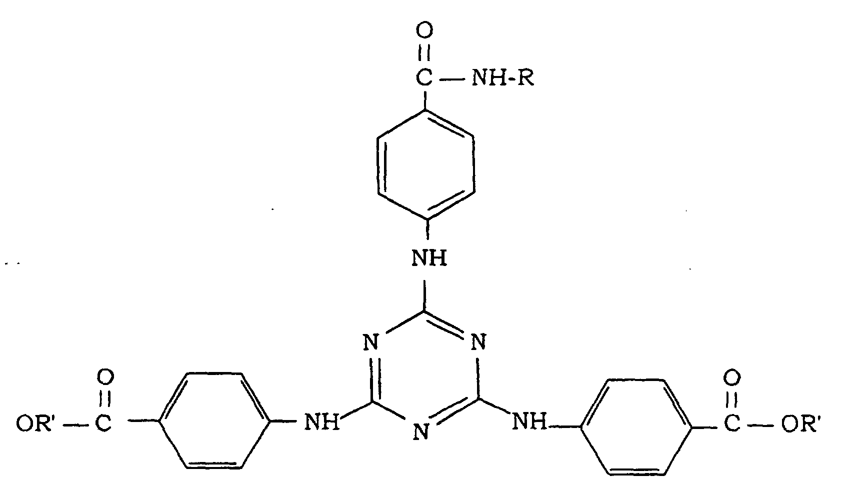 worin R' die 2-Ethylhexylgruppe bedeutet und R die *t*-Butylgruppe ist.

16. Zusammensetzung nach Anspruch 11, **dadurch gekennzeichnet, dass** das anorganische Pigment unter den Nanopigmenten der Oxide von Titan, Eisen, Zink, Zirkonium oder Cer ausgewählt ist, die gegebenenfalls mit Aluminiumoxid und/oder Aluminiumstearat umhüllt sind.

17. Kosmetische Verwendung der Zusammensetzung nach einem der Ansprüche 1 bis 16 zur Vorbeugung oder Behandlung der Anzeichen der altersbedingten oder lichtinduzierten Hautalterung.

18. Verwendung der Zusammensetzung nach einem der Ansprüche 1 bis 16 für die Herstellung eines Präparats, das zur Vorbeugung oder Behandlung der durch UV-Strahlung verursachten Hautschäden vorgesehen ist.
